# EUROPÄISCHE PATENTANMELDUNG

(11) **EP 3 047 911 A1**
(43) Veröffentlichungstag der Anmeldung: **27.07.2016**
(21) Anmeldenummer: 15152205.9
(22) Anmeldetag: 22.01.2015
(51) Int. Cl.: B03C 1/28, B03C 1/033, B03C 1/30, A61M 25/01

(54) **Separiereinrichtung zur Rückhaltung von in einem Fluid befindlichen magnetischen Partikeln sowie Schutzreinrichtung für ein Funktionselement**

(71) Anmelder: ECP Entwicklungsgesellschaft mbH, 12247 Berlin (DE)
(72) Erfinder: Er, Sami, 10785 Berlin (DE); Simon, Cornelia, 81925 München (DE); Schumacher, Jörg, 14513 Teltow (DE); Liebing, Reiner, 13187 Berlin (DE)
(74) Vertreter: Pfenning, Meinig & Partner mbB

(57) **Zusammenfassung**

Die Erfindung bezieht sich auf eine Separiereinrichtung zur Rückhaltung von in einem Fluid befindlichen magnetischen Partikeln (15, 16) mit einem Transportkanal (1, 1'), in dem das Fluid in einer Durchflussrichtung (4, 5, 6) bewegt werden kann, und mit einer Magneteinrichtung (13, 13', 13", 14, 18, 19, 20), wobei die Magneteinrichtung wenigstens einen Magneten (13, 13', 13") aufweist, der durch eine magnetisch durchlässige Feststoffschicht (14) von dem Fluid getrennt ist. Zudem bezieht sich die Erfindung auf eine Kathetereinrichtung mit einem Katheter (24), in dem eine rotierende, wenigstens teilweise aus einem magnetischen Material bestehende Welle (25) angeordnet ist, und mit einer Separiereinrichtung, die einen die rotierende Welle umgebenden Ringkörper (27) mit einem einen Magnetkörper enthaltenden Hohlraum enthält, wobei der Magnetkörper bezüglich der Flussrichtung des Fluids durch den Katheter (24) stromabwärts von einer Stelle angeordnet ist, an der die Welle (25) aus dem sie umgebenden Katheter austritt. Durch die Erfindung lassen sich somit magnetische Partikel aus einem Fluidstrom zurückhalten, um mechanische Komponenten vor Verschleiß zu schützen.

## Beschreibung

Die Erfindung liegt auf dem Gebiet des Maschinenbaus und der Mechanik sowie der Fluidtechnik und ist mit besonderem Vorteil beispielsweise in der Medizintechnik anwendbar. Konkret befasst sich die Erfindung mit der Separierung von magnetischen Partikeln aus einem Fluid, insbesondere einer Flüssigkeit.

Beim Transport von Fluiden durch Fließkanäle ist es allgemein unerwünscht, dass Partikel, die beispielsweise durch Abrieb entstehen oder auf andere Weise in den Fluidkreislauf gelangen, mit dem bewegten Fluid transportiert werden. Üblicherweise haben die transportierten Partikel keinen Nutzen und bringen lediglich Risiken mit sich, indem sie beispielsweise in bewegte Teile, wie Kugellager, Gleitlager, Motoren oder Rotoren, hineingeraten und dort zumindest weiteren Abrieb verursachen oder durch erhöhte Reibung Bewegungen bremsen oder behindern. Dies fällt umso mehr ins Gewicht, wenn die bewegten Fluidmengen klein und die Bewegungsgeschwindigkeit des Fluids langsam ist, wie beispielsweise bei Spülmittelzirkulation in Kathetern, wo üblicherweise nur Milliliter in Minuten bewegt werden. Auch sind die im Zusammenhang mit medizinischen Kathetern verwendeten bewegten Teile üblicherweise sehr empfindlich, falls unerwünschte Partikel in sie hineingeraten.

In einigen Fällen können unerwünschte Partikel durch mechanische Filter, beispielsweise Gewebe, aus dem Fluidstrom herausgefiltert werden, jedoch geht dies grundsätzlich mit einer Erhöhung des Fließwiderstandes einher.

Insbesondere bei solchen Kathetern, die zum Antrieb von Funktionselementen, beispielsweise Blutpumpen oder Blutgefäßfräsen, eine schnell rotierende Welle führen, zeigt der Abrieb des Wellenmaterials im Laufe der Zeit negative Auswirkungen. Derartige Wellen bestehen häufig aus verseilten Litzen, wobei insbesondere bei einer gebogenen Führung einer derartigen Welle und hohen Drehzahlen durch die Walkarbeit der verseilten Litzen untereinander ein verstärkter Abrieb entsteht.

Zur Rückhaltung von magnetischen Partikeln sind grundsätzlich bereits Magnetfilter bekannt. Jedoch sind diese üblicherweise für die geringen Durchflussmengen von wenigen Millilitern pro Stunde zu groß und zudem nicht für die Verwendung mit Kochsalzlösungen oder anderen aggressiven Fluiden geeignet. Membranfilter stellen üblicherweise einen zu hohen Leitungswiderstand dar und sind zu groß und zu teuer, um beispielsweise als Einwegfilter eingesetzt werden zu können. Außerdem können Membranfilter und insbesondere die sich in diesen ansammelnde Partikelmenge die Funktion beispielsweise einer flexiblen Welle erheblich stören, was bis zur Zerstörung der flexiblen Welle führen kann.

Dem vorliegenden Schutzrecht liegt vor dem Hintergrund des Standes der Technik die Aufgabe zugrunde, eine Separiereinrichtung zu schaffen, die es erlaubt, aus einem Fluidstrom magnetische Partikel zurückzuhalten, ohne den Fluidstrom zu behindern oder zu verlangsamen, wobei die Separiereinrichtung auch dazu eingerichtet sein soll, aggressiven Fluiden zu widerstehen.

Die Aufgabe wird mit den Merkmalen gemäß Patentanspruch 1 gelöst. Die Unteransprüche stellen Weiterbildungen dar. Bezüglich einer Kathetereinrichtung mit einem Katheter und einer Separiereinrichtung wird ebenfalls eine Lösung angegeben. Außerdem wird eine Schutzeinrichtung für ein Funktionselement sowie ein Kathetersystem beansprucht.

Das Schutzrecht bezieht sich demgemäß auf eine Separiereinrichtung zur Rückhaltung von in einem Fluid befindlichen magnetischen Partikeln mit einem Transportkanal, in dem das Fluid in einer Durchflussrichtung bewegt werden kann, und mit einer Magneteinrichtung, wobei die Magneteinrichtung wenigstens einen Magneten aufweist, der durch eine magnetisch durchlässige Feststoffschicht von dem Fluid getrennt ist. Vorteilhaft kann der Magnet vollständig durch die Feststoffschicht von dem Fluid isoliert, insbesondere allseitig von der Feststoffschicht umgeben sein.

Die Magneteinrichtung der Separiereinrichtung kann beispielsweise einen oder mehrere Dauermagnete oder einen oder mehrere Elektromagnete oder eine Mischung beider aufweisen, und die Anwendung eines Magnetfeldes sorgt, wenn das Fluid durch den Transportkanal strömt, dafür, dass magnetische Partikel, also beispielsweise Eisenpartikel, die magnetisiert oder unmagnetisiert sein können, in der Nähe des Magneten an der Innenwand eines Fließkanals/Fluidkanals oder unmittelbar an der Feststoffschicht des Magneten hängen bleiben. Dabei wird die Strömung des Fluids durch den Transportkanal bzw. den Fluidkanal nicht behindert. Zudem ist durch die Trennung des/der Magneten vom eigentlichen Fluid sichergestellt, dass auch bei einer hohen chemischen oder physikalischen Aggressivität des Fluids, beispielsweise bei Verwendung von Salzlösung, jedoch bei nichtmedizinischen Anwendungen auch bei der Verwendung von Säuren oder heißen Flüssigkeiten, das Material des Magneten selbst nicht beschädigt wird.

Zum Spülen der Separiereinrichtung kann beispielsweise ein Elektromagnet ausgeschaltet oder ein Permanentmagnet vorübergehend von dem Transportkanal entfernt werden. Dies hat den Vorteil, dass das Spülen der Separiereinrichtung ohne ein Entfernen der Separiereinrichtung selbst vom Transportkanal möglich ist.

Eine Ausgestaltung kann vorsehen, dass der Magnet ausschließlich mit magnetischen oder magnetisierbaren Partikeln im Fluid im Transportkanal wechselwirkt.

Der Magnet der Separiereinrichtung ist insbesondere auch separat von einem zusätzlichen Magneten oder Anker eines Pumpenantriebs und/oder eines Ventilantriebs vorgesehen, die benachbart zu der Separiereinrichtung, insbesondere bezüglich der bevorzugten Stromrichtung des Fluids abwärts von der Separiereinrichtung, vorgesehen sein können.

Eine Ausgestaltung sieht vor, dass die Separiereinrichtung einen ersten und einen zweiten Fluidanschluss aufweist, zwischen denen die Separiereinrichtung einen fluiddichten Fluidkanal ausbildet.

In diesem Fall ist innerhalb des Transportkanals unmittelbar im Rahmen der Separiereinrichtung ein Fluidkanal ausgebildet, in dem das Fluid, also beispielsweise eine Flüssigkeit, zwischen einem ersten und zweiten Fluidanschluss, also beispielsweise zwischen einem Zuflusskanal und einem Abflusskanal, bewegt wird. Der Fluidkanal kann in diesem Fall den Transportkanal bilden oder innerhalb des Transportkanals beispielsweise in Form eines Katheters ausgebildet sein.

Eine weitere vorteilhafte Ausgestaltung sieht vor, dass in dem Fluidkanal ein allseitig vom Fluid umströmbarer, mit einer magnetisch durchlässigen Feststoffschicht umhüllter Magnet angeordnet ist.

In diesem Fall ist der Magnet innerhalb des Fluidkanals angeordnet und kann dem vorbeiströmenden Fluid eine maximale Interaktionsfläche bieten. Vorteilhaft ist dabei eine entsprechende Erweiterung des Querschnitts des Fluidkanals, so dass genug Platz für das Fluid ist, um allseitig an dem Magneten vorbeiströmen zu können. Der Magnet selbst kann beispielsweise mit einer Kunststoffschicht oder auch mit einer ausreichend veredelten Metallisierung allseitig oder wenigstens an den mit dem Fluid beaufschlagten Seiten abgedeckt sein. Der Magnet sollte mit seiner Umhüllung beispielsweise durch Streben oder eine andere Halteeinrichtung innerhalb des Fluidkanals gehalten werden.

Es kann dabei vorteilhaft vorgesehen sein, dass der Magnet als Zylinder oder Quader ausgebildet ist, dessen Länge in Längsrichtung des Fluidkanals größer ist als sein Durchmesser in Querrichtung des Fluidkanals und der in einem zylindrischen Abschnitt des Fluidkanals angeordnet ist.

In diesem Fall ist sowohl der zylindrische oder im Querschnitt viereckige Abschnitt des Fluidkanals als auch der Magnet länglich ausgebildet, so dass sich für das vorbeiströmende Fluid an dem Magneten ausreichende Wechselwirkungszeiten ergeben, um die entsprechenden magnetischen Partikel zum Magneten zu ziehen und dort festzuhalten.

Zudem kann vorteilhaft vorgesehen sein, dass die Magnetfeldlinien innerhalb des Magneten quer, insbesondere senkrecht zur Strömungsrichtung des Fluids, verlaufen.

In diesem Fall wird jeweils an den Seiten des Magneten, an denen das Fluid in Längsrichtung des Fluidkanals vorbeiströmt, ein Magnetpol ausgebildet werden, der entsprechende magnetische Teile festhält. Die Magnetisierung kann allerdings auch derart ausgebildet sein, dass die Magnetpole in Längsrichtung des Fluidkanals ausgerichtet sind. Es ergibt sich dann die Hauptwechselwirkungsfläche des Magneten mit den magnetischen Partikel im Fluid an den beiden stromaufwärts und stromabwärts gelegenen Enden des Magneten.

Die Separiereinrichtung kann auch vorteilhaft derart ausgebildet sein, dass der Magnet in Durchflussrichtung eine geringere Ausdehnung aufweist als senkrecht zur Durchflussrichtung.

In diesem Fall kann der Magnet eine Scheibenform ausbilden, wobei die magnetische Scheibe senkrecht zur Fluidrichtung im Fluidkanal gestellt ist und gegebenenfalls Verwirbelungen des Fluids erzeugt, das um die Scheibe herumströmt. In diesem Fall ergibt sich ein gewisser Fließwiderstand durch den Magneten, jedoch wird durch die Verwirbelung des Fluids erreicht, dass alle im Fluid befindlichen Partikel auf dem von ihnen zurückgelegten Weg irgendwann in die unmittelbare Nähe des Magneten gelangen und dort festgehalten werden können.

Im Fall eines länglichen Magneten können im Bereich der Separiereinrichtung im Fluidstorm feststehende Verwirbelungselemente vorgesehen sein, die für eine nichtlaminare Strömung sorgen und dafür, dass die Partikel der Magneteinrichtung nahe kommen.

Eine weitere vorteilhafte Ausgestaltung sieht beispielsweise einen Ringkörper vor, der den Transportkanal umgibt, wobei der Transportkanal zur Aufnahme eines Katheters mit einem Durchflusskanal eingerichtet ist und wobei in dem Ringkörper in einem neben dem Transportkanal gelegenen Hohlraum ein Magnet angeordnet ist.

In diesem Fall kommt die Separiereinrichtung selbst nicht unmittelbar mit dem Fluid in Kontakt, sondern der Transportkanal ist derart eingerichtet, dass er einen Katheter mit einem Fluidkanal aufnehmen kann. Dies hat den Vorteil, dass die Separiereinrichtung ohne eine Unterbrechung eines Fluidkanals, also beispielsweise auch ohne Unterbrechung des Fluidflusses, eingerichtet und abgebaut werden kann. Dazu kann beispielsweise vorgesehen sein, dass der Ringkörper in Umfangsrichtung einstückig ausgebildet ist. Es kann jedoch auch vorgesehen sein, dass der Ringkörper in Umfangsrichtung wenigstens einmal unterbrochen und insbesondere zum Aufstecken auf einen Katheter aufklappbar ist.

In diesem Fall ist in besonders einfacher Weise die Anwendung und Einrichtung der Separiereinrichtung an einem Katheter möglich, indem die Separiereinrichtung mit dem Ringkörper einfach aufgeklappt und auf den Katheter aufgeschoben wird. Auch das Entfernen der Separiereinrichtung ist entsprechend einfach. Allerdings kann sich durch diese Bauform die Baugröße der Separiereinrichtung gegenüber einer Separiereinrichtung, die einen im Fluidkanal befindlichen Magneten aufweist, etwas vergrößern.

Eine weitere vorteilhafte Ausgestaltung sieht vor, dass der Fließkanal im Bereich der Magneteinrichtung einen größeren Querschnitt aufweist als in einem in Richtung des Fluids vor dem Bereich der Magneteinrichtung angeordneten Bereich.

Durch diese Bauform wird, gleich ob die Separiereinrichtung einen den Transportkanal umgebenden Ringkörper mit einem Magneten oder einen im Fluidkanal selbst befindlichen Magneten aufweist, die Fluidströmung im Bereich der Separiereinrichtung durch den vergrößerten Querschnitt verlangsamt, so dass die magnetischen Partikel mit einer vergrößerten Wahrscheinlichkeit durch den Magneten angezogen und festgehalten werden können. Außerdem wird auf diese Weise sichergestellt, dass durch die separierten Partikel nicht der Fluidkanal verstopft bzw. der Fluidstrom behindert wird. Der Querschnitt des Fluidkanals kann unmittelbar vor der Separiereinrichtung, jedoch auch alternativ oder zusätzlich unmittelbar hinter der Separiereinrichtung gegenüber dem Bereich der Separiereinrichtung verringert sein. Dabei kann der Querschnitt im Bereich der Separiereinrichtung beispielsweise wenigstens doppelt, insbesondere wenigstens dreifach oder fünffach so groß sein wie unmittelbar vor der Separiereinrichtung, und beispielsweise auch mindestens zwei-, drei- oder fünfmal so hoch wie unmittelbar hinter der Separiereinrichtung in Fließrichtung des Fluids. Es kann aber der Fluidkanal auch so ausgebildet werden, dass der Magnet die Partikel in einen Hohlraum bzw. e Reservoir befördert, so dass diese nicht den Fluidstrom behindern.

Das vorliegende Schutzrecht bezieht sich außer auf eine Separiereinrichtung auch auf eine Kathetereinrichtung mit einem Katheter, in dem eine rotierende, wenigstens teilweise aus einem magnetischen Material bestehende Welle angeordnet ist, und mit einer Separiereinrichtung, die einen die rotierende Welle umgebenden Ringkörper mit einem einen Magnetkörper enthaltenden Hohlraum enthält, wobei der Magnetkörper bezüglich der Flussrichtung des Fluids durch den Katheter stromabwärts von einer Stelle angeordnet ist, an der die Welle aus dem sie umgebenden Katheter austritt.

Hierdurch wird deutlich, dass die Separiereinrichtung mit einer entsprechenden Kathetereinrichtung besonders in dem Fall vorteilhaft einsetzbar ist, dass der Abrieb einer rotierenden Welle innerhalb eines Fluidstroms separiert werden muss. Beispielsweise in medizinischen Einrichtungen werden derartige Katheter mit schnell rotierenden Wellen zum Antrieb von Funktionselementen wie Fräsen für Blutgefäße oder Herzpumpen verwendet, so dass der auftretende Abrieb des Wellenmaterials für die sehr fein und exakt, jedoch auch empfindlich gebauten Funktionselemente, beispielsweise für entsprechende Gleitlager, schädlich ist. Besonders in diesem Zusammenhang ist es daher wichtig, den Abrieb der Welle, die üblicherweise aus verseilten Litzen einer Eisenlegierung besteht, aufzufangen.

Außer auf eine Separiereinrichtung der oben beschriebenen Art sowie eine Kathetereinrichtung bezieht sich das vorliegende Schutzrecht weiter auf eine Schutzeinrichtung für ein Funktionselement, das mit einem strömenden Fluid in Verbindung steht, wobei entlang eines Strömungskanals für das Fluid, insbesondere eines Katheters, von dem Funktionselement beabstandet und insbesondere von diesem separiert eine Separiereinrichtung zur Rückhaltung von im Fluid befindlichen Partikeln mit wenigstens einem Magnetelement, insbesondere eine Separiereinrichtung der oben beschriebenen Art, vorgesehen ist.

Die Separiereinrichtung kann vorteilhaft bezüglich der vorherrschenden Strömungsrichtung des Fluids stromaufwärts von dem Funktionselement vorgesehen sein, jedoch können die beiden genannten Elemente auch einfach hintereinander, insbesondere voneinander beabstandet, beispielsweise auch baulich voneinander getrennt, zum Beispiel in Form von zwei separaten Bauelementen mit verschiedenen Gehäusen, vorgesehen sein.

Das Funktionselement kann frei von magnetischen oder magnetisch wirkenden Elementen sein und beispielsweise insgesamt unmagnetisch sein. Es kann ein oder mehrere Kugellager und/oder Gleitlager enthalten. Das Funktionselement kann beispielsweise auch eine Dichtfläche aufweisen, die vor Partikeln geschützt werden soll.

Das Funktionselement kann auch magnetische Bauteile enthalten, wie beispielsweise einen Antriebsmagneten eines Rotors oder eines translatorischen Antriebs oder einen Antriebsmagneten eines Magnetventils. Das Magnetelement der Separiereinrichtung kann ein von den magnetischen Bauteilen des Funktionselementes getrennter Magnet sein oder eine Funktionsfläche eines magnetischen Bauelementes, die ausschließlich die Funktion der Partikelseparation hat, wobei andere Funktionsflächen des magnetischen Bauelementes andere Funktionen des Funktionselementes, wie beispielsweise eine Antriebsfunktion, ausüben können. In diesem letztgenannten Fall kann das Magnetelement der Separiereinrichtung mit einem magnetischen Bauelement des Funktionselementes kombiniert, mit diesem zusammengefügt, zusammengefasst und insbesondere auch in einem Gehäuse zusammengefasst sein. Die Funktionsfläche der Separiereinrichtung kann somit Partikel, insbesondere magnetische und/oder magnetisierbare Partikel, einfangen und binden, bevor sie zu dem Funktionselement gelangen können.

Ein zusätzlicher Aspekt betrifft ein Funktionselement, das mit einer Separiereinrichtung, insbesondere gemäß dem vorliegenden Schutzrecht, verbunden ist, insbesondere ein Ventil, das ein zwischen zwei Endstellungen antreibbares Verschlusselement aufweist, wobei in das Verschlusselement ein oder zwei Anker aus einem magnetischen oder magnetisierbaren Material integriert ist/sind oder aus einem Material mit einem besonders geringen magnetischen Widerstand und wobei ein Magnet der Separiereinrichtung mit dem Verschlusselement kombiniert, insbesondere mit diesem fest verbunden, vorteilhaft in dieses mit integriert ist.

Die Erfindung wird nachfolgend anhand von Ausführungsbeispielen in Figuren einer Zeichnung gezeigt und erläutert.

Dabei zeigt:
- FIG. 1: in einem Längsschnitt eine Separiereinrichtung mit einem Transportkanal, der als Fluidkanal ausgebildet ist und in dem ein Magnet von einem Fluid umströmt ist,
- FIG. 2: eine Kathetereinrichtung mit einer rotierenden Welle und einer Separiereinrichtung in einem Längsschnitt,
- FIG. 3: einen Querschnitt der in FIG. 2 gezeigten Vorrichtung,

- FIG. 4: eine Magnetvorrichtung, bei der der vom Fluid umströmte Magnet quer zur Längsrichtung des Fluidkanals magnetisiert ist,
- FIG. 5: einen Magneten, der in seiner Längsrichtung und in Längsrichtung des Fluidkanals magnetisiert ist,
- FIG. 6: ein Ventil, das mit einer Separiereinrichtung verbunden ist,
- FIG. 7: ein weiteres mit einer Separiereinrichtung verbundenes Ventil,
- FIG. 8: eine Antriebseinheit für ein Funktionselement, das mittels einer in einem Katheter rotierenden Welle antreibbar ist,
- FIG. 9: eine Abwandlung einer Antriebseinheit nach FIG. 8,
- FIG. 10 und FIG. 11: jeweils weitere Gestaltungsformen von Antriebseinrichtungen für in einem Katheter rotierende Wellen sowie
- FIG. 12: eine Abwandlung einer Antriebseinheit nach FIG. 9.

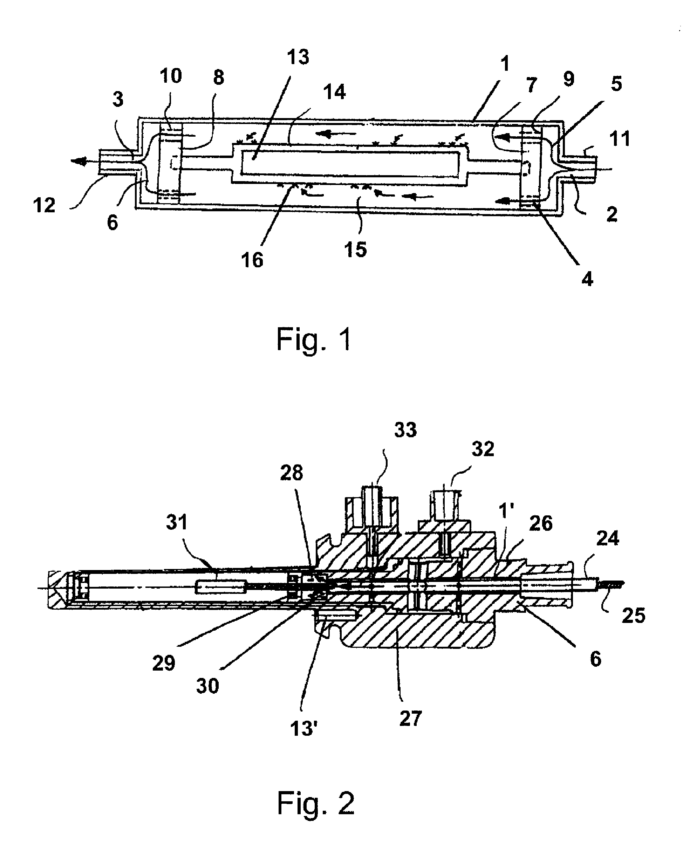

Die FIG. 1 zeigt in einem Längsschnitt einen Transportkanal 1, der unmittelbar als Fluidkanal ausgebildet ist, und ein Fluid, beispielsweise in Form einer Kochsalzlösung, führt. Das Fluid tritt an einer Zuflussöffnung 2 in den Transportkanal 1 ein und tritt an der Abflussöffnung 3 aus diesem aus. Die Flussrichtungen sind durch Pfeile 4, 5, 6 bezeichnet. Am stromaufwärts gelegenen Ende des Transportkanals 1 ist eine Halterung 7 für eine Magneteinrichtung vorgesehen, während am stromabwärts gelegenen Ende eine weitere Halterung 8 für eine Magneteinrichtung vorgesehen ist. Die Halterungen 7, 8 können beispielsweise als Tragsterne mit Fluiddurchlassöffnungen 9, 10 ausgebildet sein. Der Querschnitt der Durchflussöffnungen 9, 10 sollte dabei so groß sein, dass die Halterungen 7, 8 keinen nennenswerten Strömungswiderstand für das Fluid darstellen.

Die Zuflussöffnung 2 kann ebenso wie die Abflussöffnung 3 jeweils mit einem Katheter verbunden sein, der beispielsweise auf einen Anschlussstutzen 11, 12 aufgeschoben sein kann.

Im Inneren des Transportkanals ist eine Magneteinrichtung mit einem Permanentmagneten 13 angeordnet, der von einer Umhüllung 14 allseits umgeben ist, die den Magneten vor dem Einfluss eines korrosiven Fluids schützt. Die Umhüllung kann beispielsweise als Kunststoffumhüllung, Lackierung oder auch als Metallisierung, das heißt eine metallische Beschichtung aus einem edlen Metall, ausgeführt sein.

Die Strömung des Fluids durch den Transportkanal 1 wird keine streng laminare Strömung sein, sondern gewisse Verwirbelungen aufweisen. In jedem Fall werden die Partikel 15, 16, die als magnetische Partikel beispielsweise durch Abrieb magnetischer Teile im Fluidkreislauf vorhanden sind, von bestimmten Bereichen des Magneten angezogen. Es kann auch durch zusätzliche Verwirbelungselemente im Transportkanal 1 dafür gesorgt werden, dass die Strömung des Fluids verwirbelt wird, so dass die Wahrscheinlichkeit, dass in dem Fluid transportierte Partikel in die Nähe des Magneten gelangen, erhöht wird. Unter dem Begriff "magnetische Partikel" werden dabei alle Partikel verstanden, die durch einen Magneten angezogen werden, insbesondere, aber nicht nur, ferromagnetische Partikel.

Sind die Partikel einmal in einen Einfangbereich des Magneten gelangt, so werden sie dort festgehalten und aus dem Fluidfluss zurückgehalten. Die in der FIG. 1 gezeigte Separiereinrichtung kann beispielsweise als Einmal-Separiereinrichtung verwendet und nach der Verwendung entsorgt werden. In diesem Fall können die separierten Metallpartikel 15, 16 am Magneten 13 verbleiben. Es kann auch vorgesehen sein, dass der Magnet 13 als Elektromagnet ausgebildet ist oder von außerhalb des Transportkanals durch eine Magneteinrichtung magnetisiert wird. In diesen beiden Fällen kann die Magnetisierung des Magneten 13 vorübergehend aufgehoben werden, um den Transportkanal und die äußere Oberfläche des Magneten 13, 14 spülen zu können und die magnetischen Partikel 15, 16 zu entfernen. In diesem Fall kann beispielsweise ein anderer Katheter an den Anschlussstutzen 12 angeschlossen werden, der das zum Spülen benutzte Fluid mitsamt den Partikeln in einen Auffangbehälter leitet.

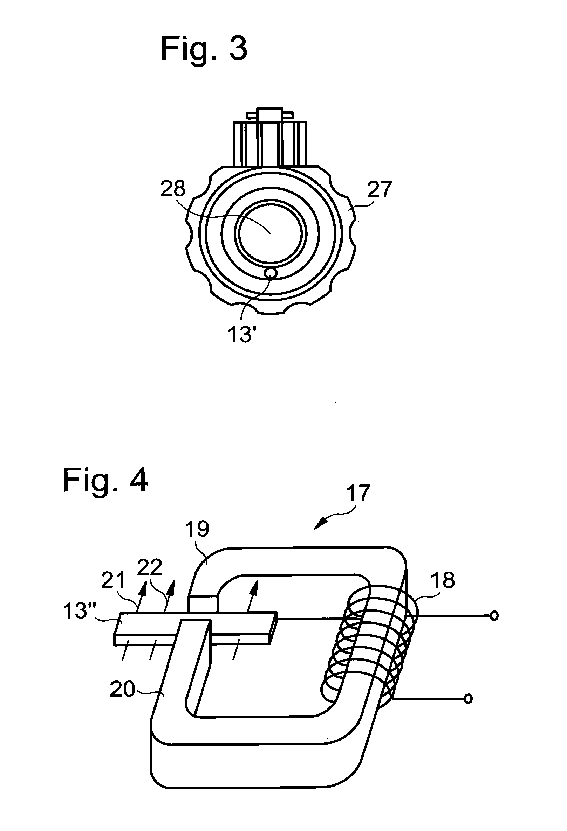

Der Magnet 13" kann, wie in der FIG. 4 näher gezeigt ist, beispielsweise durch eine äußere Magnetisierungseinrichtung 17 mit einem Elektromagnetteil 18 sowie Polschuhen 19, 20 magnetisiert werden, so dass seine Magnetisierungsrichtung entlang den in der FIG. 4 gezeigten Pfeilen 21, 22 quer zur Längsrichtung des Transportkanals verläuft (vorausgesetzt ist, dass der in der FIG. 4 gezeigte Magnet für eine Vorrichtung verwendet wird, wie sie in der FIG. 1 dargestellt ist). Zum Spülen kann dann der Elektromagnet 18 einfach abgeschaltet oder seine Wirkung wenigstens teilweise umgekehrt werden, um die Restmagnetisierung des Magneten 13 zu beseitigen.

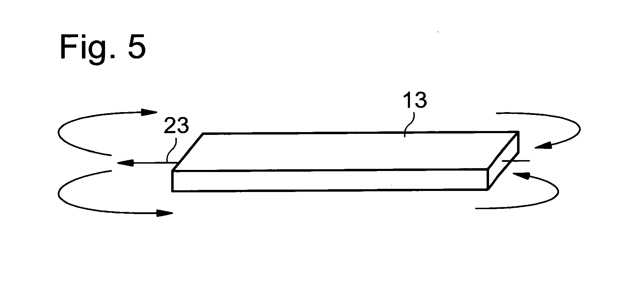

In der FIG. 5 ist eine weitere Bauform eines Magneten dargestellt, wobei seine äußere geometrische Form der des in der FIG. 1 dargestellten Magneten entspricht, wobei die Magnetisierung, angedeutet durch den Pfeil 23, in der Längsrichtung des Magneten 13 verläuft.

Metallische Partikel würden sich bei Verwendung eines derartigen Magneten eher an den beiden axialen Enden ansammeln als an den Längsseiten wie bei einem in der FIG. 4 gezeigten quer zur Längsrichtung magnetisierten Magneten.

Die FIG. 2 zeigt eine Kathetereinrichtung mit einem Katheter 24, in dem eine rotierende metallische Welle 25 geführt ist. Mit dem Bezugszeichen 26 ist in der FIG. 2 eine Katheterhalterung bezeichnet, die einen Transportkanal 1' aufweist. Das Bezugszeichen 27 bezeichnet ein Gehäuse, das die Katheterhalterung 26 umgibt und einen Ringkörper bildet, der einen Hohlraum aufweist, in dem ein Magnet 13' angeordnet ist. Innerhalb des Gehäuses 27 tritt die Welle 25 aus dem Katheter 24 aus. Der Katheter 24 tritt aus der Katheterhalterung 26 aus oder endet an einem Ende der Katheterhalterung 26. In jedem Fall kann das in dem Katheter 24 befindliche Fluid, das langsam entlang der Welle 25 als Spül- und Schmierflüssigkeit strömt, in einen am Ende des Katheters 24 gebildeten Fluidkanal 28 austreten, der einen wesentlich größeren Querschnitt aufweist als der freie Querschnitt des Katheters 24, der bereits durch die in diesem geführte Welle 25 verringert ist. Der Fluidkanal 28 befindet sich stromaufwärts eines mechanischen Lagers 29, das als Gleitlager ausgeführt sein kann, und in unmittelbarem Einflussbereich des Magneten 13'. Der Magnet 13' ist als Permanentmagnet ausgebildet, kann jedoch auch als Elektromagnet ausgeführt sein.

Im Bereich des Fluidkanals 28 sammeln sich die magnetischen Partikel 30 an der Wand des Kanals, die dem Magneten 13' zugewandt ist. Auf diese Weise werden die magnetischen Partikel aus dem Fluid zurückgehalten und gelangen nicht zu dem Lager 29.

Der weitere Verlauf der Welle 25 ist nicht dargestellt, jedoch können im weiteren Verlauf hinter der Anschlusskupplung 31 weitere mechanisch funktionierende Teile, wie beispielsweise durch die Welle angetriebene Pumpen oder Fräsen, vorgesehen sein, die vor dem Einfluss der magnetischen Partikel geschützt werden müssen. Das Gehäuse 27 beherbergt außer der Katheterhalterung 26 noch eine Spüleinrichtung mit Anschlussstutzen 32, 33 für ein Spülfluid, um den Katheter 24 zu spülen.

Der Magnet 13' kann zum Entfernen der eingefangenen magnetischen Partikel 30 aus dem Gehäuse 27 abgezogen werden, so dass dann die magnetischen Partikel weggespült werden können. Dies sollte außerhalb der Betriebszeit der Welle und der entsprechenden Lager und Funktionselemente geschehen, um diese zu schonen. Handelt es sich bei dem Magneten 13' um einen Elektromagneten, so kann dieser zum Spülen einfach vorübergehend ausgeschaltet werden.

In der FIG. 3 ist ein Querschnitt durch die Katheteranordnung aus der FIG. 2 gezeigt, mit dem Gehäuse 27, dem Transportkanal 28 in dem Bereich hinter dem Ende des Katheters 24 sowie dem in einem Hohlraum des Gehäuses 27 befindlichen Magneten 13'.

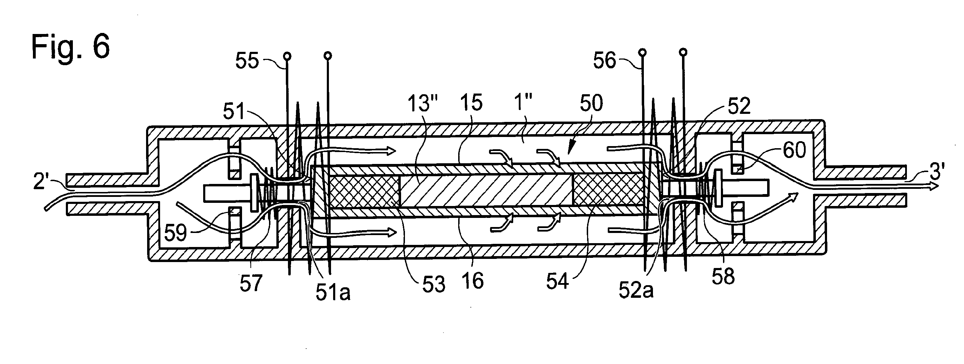

FIG. 6 zeigt ein Magnetventil mit einem Transportkanal 1", der von einem Fluid zwischen einer Zuflussöffnung 2' und einer Abflussöffnung 3' durchströmt wird. Ein Verschlusskörper 50 ist innerhalb des Transportkanals 1" zwischen einer ersten Verschlussstellung und einer zweiten Verschlussstellung antreibbar, wobei in der ersten Verschlussstellung eine erste Verschlussfläche 51 eine Ventilöffnung 51a verschließt, während in der zweiten Verschlussstellung eine Verschlussfläche 52 eine Ventilöffnung 52 a verschließt.

In den Verschlusskörper 50 sind zwei Ankerkörper 53, 54 integriert, die durch das Magnetfeld von zwei Ventilantriebsspulen 55, 56 antreibbar sind. Axial zwischen den Ankerkörpern 53, 54 ist fluchtend mit diesen der Magnet 13" der Separiereinrichtung angeordnet. Die Ankerkörper sind mit dem Magnetkörper 13" mit einer gemeinsamen Feststoffumhüllung versehen.

Haltefedern 57, 58 halten den Verschlusskörper in Abwesenheit einer Erregung der Ventilantriebsspulen in einer Mittelstellung, in der das Ventil geöffnet ist. Zur Führung des Verschlusskörpers 50 sind zwei Gleitlager 59, 60 an den Enden des Ventilgehäuses vorgesehen.

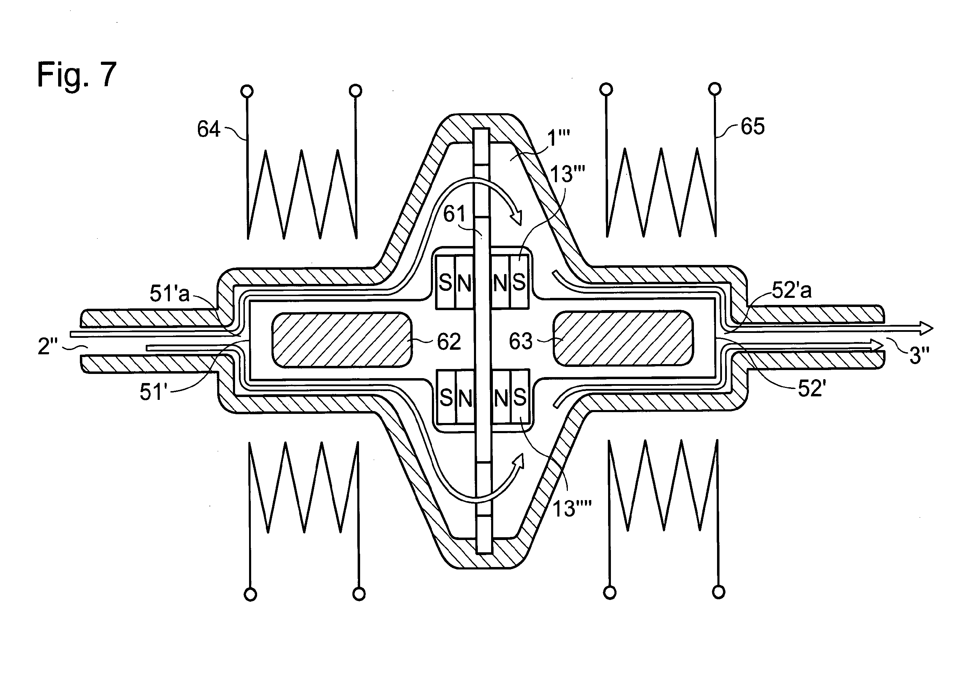

FIG. 7 zeigt ein Ventil mit einer Zuflussöffnung 2", einer Abflussöffnung 3" und einem Verschlusskörper 50'. Der Verschlusskörper 50' ist innerhalb des Transportkanals 1"' zwischen einer ersten Verschlussstellung und einer zweiten Verschlussstellung antreibbar, wobei in der ersten Verschlussstellung eine erste Verschlussfläche 51' eine Ventilöffnung 51a' verschließt, während in der zweiten Verschlussstellung eine Verschlussfläche 52' eine Ventilöffnung 52a' verschließt. Der Verschlusskörper 50' ist mittels einer elastischen, durchlässigen Scheibe 61 in dem Gehäuse des Ventils gelagert und in einer geöffneten Mittelstellung gehalten. Die Scheibe 61 trägt Separiermagnete 13"', 13"", die in dem Verschlusskörper 50' mit Ventilantriebsankern 62, 63 verbunden und gemeinsam mit diesen von einer Schutzschicht umhüllt sind.

Die Ventilantriebsanker 62, 63 sind im Feld der Spulen 64, 65 antreibbar. Partikel können sich im Transportkanal an den Separiermagneten auf der Schutzschicht anlagern und werden dort festgehalten.

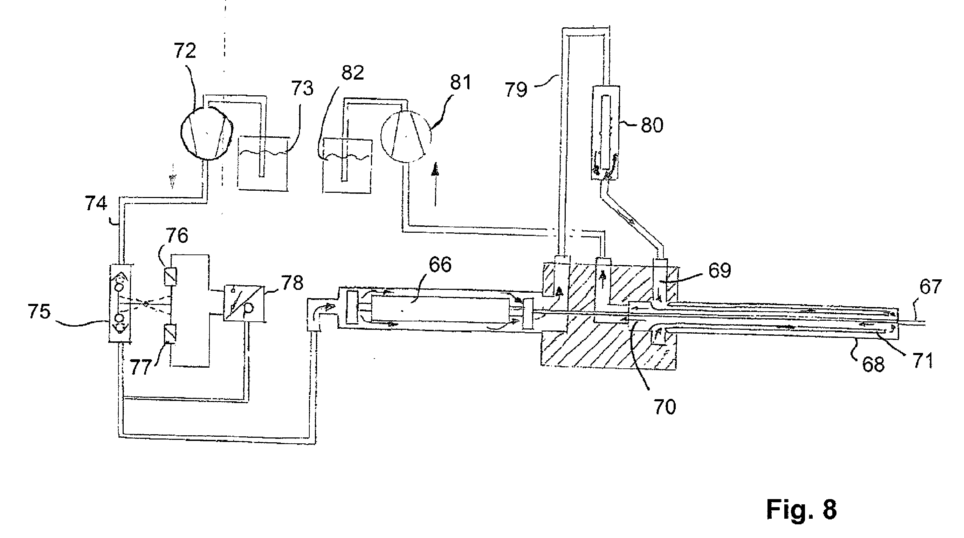

Die FIG. 8 zeigt eine Antriebseinrichtung mit einem rotierend antreibbaren Antriebsanker 66, der eine rotierende Welle 67 in einem Katheter 68 antreibt. Innerhalb des Katheters 68 sind ein Zuströmkanal 69 radial außen und ein Rückstromkanal 70 radial innen, konzentrisch zueinander und zur Außenhülle des Katheters angeordnet. Der Zuströmkanal 69 und der Rückströmkanal 70 sind durch eine schlauchartige Trennwand 71 voneinander getrennt.

Durch eine volumengesteuerte Schlauchpumpe 72 wird eine Spülflüssigkeit von einem Reservoir 73 durch eine Kanüle 74 und ein Ventil 75 gepumpt. Zwei Magnete 76 und 77 dienen dem Antrieb des Ventils und werden mittels eines Druckschalters 78 angesteuert, mit dem Ziel, einen gleichbleibenden Druck im Zuströmkanal 69 aufrechtzuerhalten. Hierzu wird das Fluid durch das Ventil 75 und durch das Gehäuse des Antriebsankers 66 den Transportkanal 79 und durch die Separiereinrichtung 80 geleitet, wo aktiv Partikel aus dem Fluid ausgefiltert werden. Die Separiereinrichtung 80 kann so aufgebaut sein wie die in der FIG. 1 gezeigte Separiereinrichtung. Von dort strömt das Fluid in den Katheter 68 durch den Zuströmkanal 69 radial außen und den Rückstromkanal 70 radial innen sowie von dort zu einer Schlauchpumpe 81, die das Fluid ansaugt und in das Reservoir 82 leitet. Die Schlauchpumpe 81 kann jedoch auch der Rückspülung dienen und zu diesem Zweck derart betrieben werden, dass sie das Fluid zum Rücklaufkanal 70 und von dort über den Zuströmkanal 69, durch die Separiereinrichtung zurück zum Ventil 75 in das Reservoir 73 fördert, um beispielsweise die eingefangenen Partikel von der Separiereinrichtung zu entfernen.

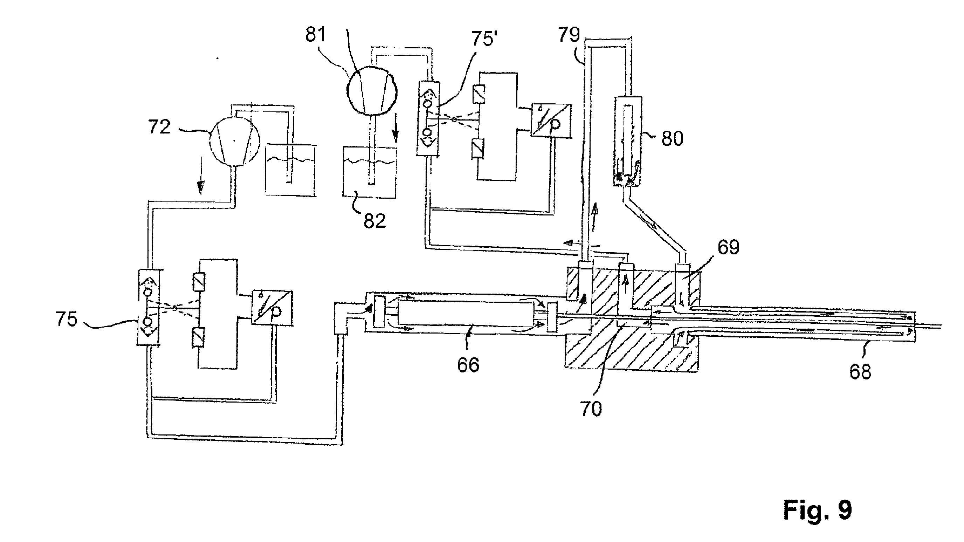

Die FIG. 9 zeigt einen Aufbau ähnlich der aus FIG. 8, wobei zusätzlich zu dem Ventil 75 vor dem Antriebsanker 66 und hinter der Schlauchpumpe 72 ein zweites Ventil 75' zwischen dem Rückströmkanal 70 und der Rückströmpumpe 80 angeordnet ist. Während FIG.8 bei Spülsystemen Anwendung findet, in denen durch Anlagenkomponenten kein ungewollter Unterdruck im Rücklauf erzeugt wird, ist es möglich, FIG.9 auch bei Spülsystemen einzusetzen, in denen im Rücklauf ein ungewollter Unterdruck entsteht (z. B. durch Wicklungsrichtung einer biegsamen Welle). Dieser Unterdruck wird vom Sensor erkannt, der dann durch Schließen des Ventils 75' nach unten dafür sorgt, dass kein Medium aus Behälter 82 über die Pumpe 81 in den Spülkreislauf kommt. Die Separiereinrichtung ist somit zwischen zwei Ventilen und auch zwischen zwei Fluidfördereinrichtungen angeordnet, von denen wenigstens eines, insbesondere beide, bezüglich der Förderrichtung des Fluides umschaltbar sind, um die Strömungsrichtung umzukehren.

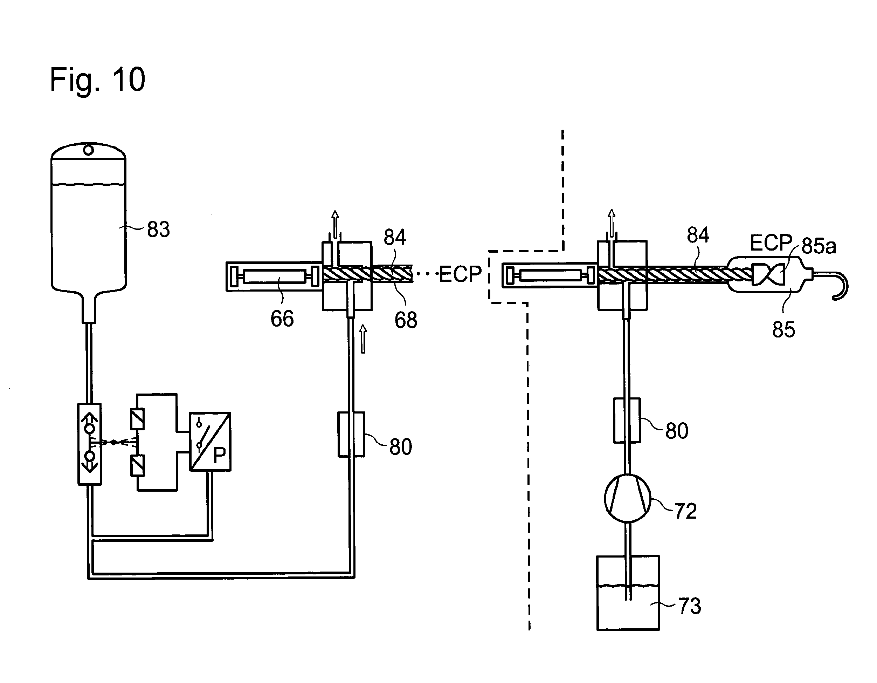

Bei dem Aufbau gemäß FIG. 10 ist gegenüber dem Aufbau in FIG. 8 lediglich eine Schlauchpumpe 72 durch ein Reservoir 83 ersetzt, das eine Schwerkraftspülung erlaubt, indem das Fluid durch die Schwerkraftdurch das Ventil 75 und weiter zum Katheter 68 fließt. Die rotierende Welle 84 innerhalb des Katheters 68 weist durch ihren verseilten Aufbau auf der Basis von verseilten Litzen eine gewendelte Außenstruktur auf, die ihr selbst bei Rotation eine Pumpwirkung in Richtung vom Antriebsanker 66 weg verleiht. Auf der rechten Seite der FIG. 10, rechts von der gestrichelten Linie 85, ist für den Zufluss von Fluid zum Katheter 68 eine andere Variante mit einer volumengesteuerten Schlauchpumpe 72 und einem Reservoir 73 dargestellt. Die Schlauchpumpe fördert dort das Fluid zum Inneren des Katheters, der beispielsweise in den Körper eines Patienten hineinführt und dort an einer Herzpumpe 85 mit einem Rotor 85a endet. Die Herzpumpe kann beispielseise radial komprimierbar bzw. insgesamt besonders anfällig für Partikel sein, die hineingeraten, Von dort strömt das Fluid dann zurück. Eine Separiereinrichtung 80 kann jeweils in Strömungsrichtung vor dem Katheter 68 zwischen diesem und der Fördereinrichtung 73, 83, insbesondere jedenfalls vor der Herzpumpe 85, vorgesehen sein.

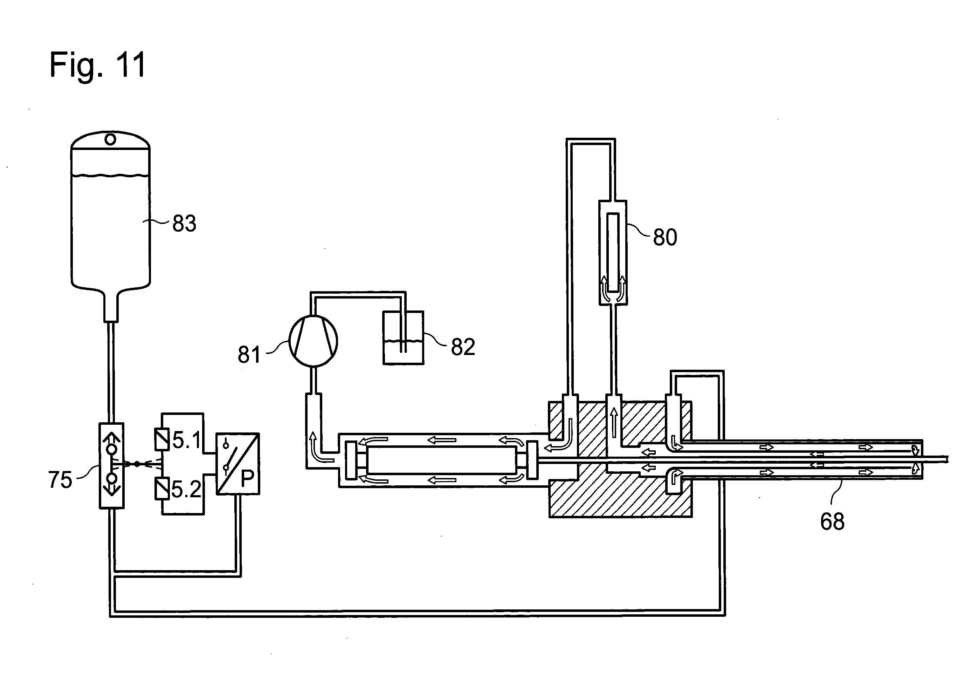

Die FIG. 11 zeigt eine Konstellation ähnlich der aus FIG. 9, wobei anstelle der Schlauchpumpe 72 eine Schwerkraftförderung 83 vorgesehen ist, wobei das Fluid im Normalbetrieb von dort über das Ventil in den Katheter 68 und dort zunächst durch den Zuströmkanal 69 radial außen in den Rückstromkanal 70 radial innen sowie von dort zu einer Schlauchpumpe 81 strömt, die das Fluid ansaugt und in das Reservoir 82 leitet. Zwischen dem Rückstromkanal 70 und der Schlauchpumpe 81 passiert das Fluid zunächst die Separiereinrichtung 80, die zwischen dem Rückstromkanal und dem Gehäuse des Antriebsankers 66 angeordnet ist. Danach strömt das Fluid am Antriebsanker 66 vorbei zur Schlauchpumpe 81. Die Lagerung des Antriebsankers kann relativ unempfindlich sein, so dass die Durchströmrichtung des Fluids dort von untergeordneter Bedeutung ist. Wichtig ist besonders, dass das Gehäuse des Antriebsankers mit dem Fluid versorgt ist, um eine gute Schmierung zu gewährleisten. Die gewählte Anordnung stellt darüber hinaus sicher, dass magnetische Abriebpartikel der rotierenden Welle 84 in diesem Fall nicht die Lager des Antriebsankers beschädigen können.

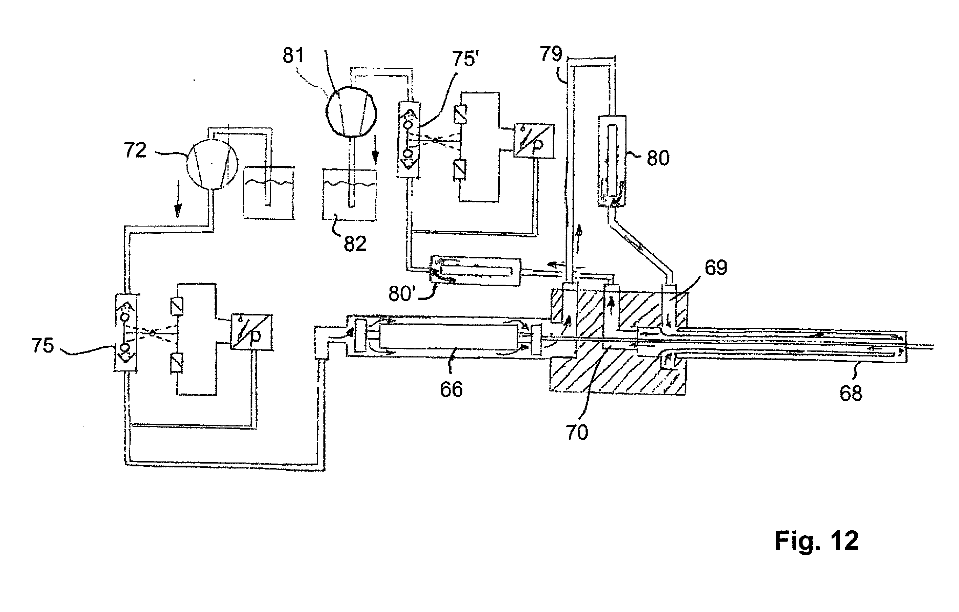

Die FIG. 12 zeigt einen Aufbau ähnlich der FIG. 9, wobei eine weitere Separiereinrichtung 80'dafür sorgt, dass die Funktion der Dichtflächen des Ventils 75' nicht durch anhaftende Partikel beeinträchtigt wird.

Die Erfindung erlaubt, insbesondere bei medizinischen Anwendungen, jedoch auch bei anderen Anwendungen, magnetische Partikel aus einem Fluidstrom mithilfe von Magneteinrichtungen zurückzuhalten, wobei die Magnete der Magneteinrichtungen vor korrosiven Wirkungen des Fluids geschützt sind.

## Patentansprüche

1. Separiereinrichtung zur Rückhaltung von in einem Fluid befindlichen magnetischen Partikeln (15, 16) mit einem Transportkanal (1, 1'), in dem das Fluid in einer Durchflussrichtung (4, 5, 6) bewegt werden kann, und mit einer Magneteinrichtung (13, 13', 13", 14, 18, 19, 20), wobei die Magneteinrichtung wenigstens einen Magneten (13, 13', 13") aufweist, der durch eine magnetisch durchlässige Feststoffschicht (14) von dem Fluid getrennt ist.

2. Separiereinrichtung nach Anspruch 1, **dadurch gekennzeichnet, dass** der Magnet ausschließlich mit magnetischen oder magnetisierbaren Partikeln im Fluid im Transportkanal wechselwirkt.

3. Separiereinrichtung nach Anspruch 1 oder 2,
***gekennzeichnet durch***
einen ersten und einen zweiten Fluidanschluss (11, 12), zwischen denen die Separiereinrichtung einen fluiddichten Fluidkanal ausbildet.

4. Separiereinrichtung nach Anspruch 1, 2 oder 3,
***dadurch gekennzeichnet, dass***
in dem Fluidkanal ein allseitig vom Fluid umströmbarer, mit einer magnetisch durchlässigen Feststoffschicht (14) umhüllter Magnet (13, 13") angeordnet ist.

5. Separiereinrichtung nach Anspruch 4,
***dadurch gekennzeichnet, dass***
der Magnet (13, 13") als Zylinder oder Quader ausgebildet ist, dessen Länge in Längsrichtung des Fluidkanals größer ist als sein Durchmesser, und der in einem zylindrischen Abschnitt des Fluidkanals angeordnet ist.

6. Separiereinrichtung nach Anspruch 5,
***dadurch gekennzeichnet, dass***
die Magnetfeldlinien innerhalb des Magneten (13, 13") quer, insbesondere senkrecht zur Strömungsrichtung des Fluids, verlaufen.

7. Separiereinrichtung nach Anspruch 4,
***dadurch gekennzeichnet, dass***
der Magnet (13, 13', 13") in Durchflussrichtung eine geringere Ausdehnung aufweist als senkrecht zur Durchflussrichtung.

8. Separiereinrichtung nach Anspruch 1, 2 oder 3,
***gekennzeichnet durch***
einen Ringkörper (27), der den Transportkanal (1') umgibt, wobei der Transportkanal zur Aufnahme eines Katheters (24) mit einem Durchflusskanal eingerichtet ist und wobei in dem Ringkörper (27) in einem neben dem Transportkanal gelegenen Hohlraum ein Magnet (13') angeordnet ist.

9. Separiereinrichtung nach Anspruch 8,
***dadurch gekennzeichnet*, *dass***
der Ringkörper (27) in Umfangsrichtung einstückig ausgebildet ist.

10. Separiereinrichtung nach Anspruch 8,
***dadurch gekennzeichnet, dass***
der Ringkörper (27) in Umfangsrichtung wenigstens einmal unterbrochen und insbesondere zum Aufstecken auf einen Katheter (24) aufklappbar ist.

11. Separiereinrichtung nach Anspruch 1 oder einem der folgenden,
***dadurch gekennzeichnet, dass***
der Fließkanal im Bereich der Magneteinrichtung (13, 13', 13", 14, 18, 19, 20) einen größeren Querschnitt aufweist als in einem in Flussrichtung des Fluids vor dem Bereich der Magneteinrichtung angeordneten Bereich.

12. Kathetereinrichtung mit einem Katheter (24), in dem eine rotierende, wenigstens teilweise aus einem magnetischen Material bestehende Welle (25) angeordnet ist, und mit einer Separiereinrichtung, die einen die rotierende Welle umgebenden Ringkörper (27) mit einem einen Magnetkörper (13') enthaltenden Hohlraum enthält, wobei der Magnetkörper bezüglich der Flussrichtung des Fluids durch den Katheter stromabwärts von einer Stelle angeordnet ist, an der die Welle (25) aus dem sie umgebenden Katheter (24) austritt.

13. Schutzeinrichtung für ein Funktionselement, das mit einem strömenden Fluid in Verbindung steht, **dadurch gekennzeichnet, dass** entlang eines Strömungskanals für das Fluid, insbesondere eines Katheters, von dem Funktionselement beabstandet und insbesondere von diesem separiert, eine Separiereinrichtung zur Rückhaltung von im Fluid befindlichen Partikeln mit wenigstens einem Magnetelement, insbesondere eine Separiereinrichtung nach einem der Patentansprüche 1 bis 11, vorgesehen ist.

14. Kathetersystem, enthaltend eine Separiereinrichtung nach einem der Ansprüche 1 bis 11 und/oder eine Schutzeinrichtung nach Anspruch 13, **dadurch gekennzeichnet, dass** zumindest ein elektrisches Element zur Steuerung eines Funktionselements und/oder zur Magnetsteuerung von dem übrigen Kathetersystem separierbar ist.
